# EUROPÄISCHE PATENTSCHRIFT

(11) **EP 0 814 778 B1**
(45) Veröffentlichungstag und Bekanntmachung des Hinweises auf die Patenterteilung: **13.06.2001**
(21) Anmeldenummer: 96906751.1
(22) Anmeldetag: 07.03.1996
(51) Int. Cl.: A61K 9/16, A61K 9/50

(54) **POLYPEPTID-ENTHALTENDE PHARMAZEUTISCHE DARREICHUNGSFORMEN IN FORM VON MIKROPARTIKELN UND VERFAHREN ZU DEREN HERSTELLUNG**
POLYPEPTIDE-CONTAINING PHARMACEUTICAL FORMS OF ADMINISTRATION IN THE FORM OF MICROPARTICLES AND METHOD FOR THE PREPARATION THEREOF
FORMES GALENIQUES PHARMACEUTIQUES CONTENANT UN POLYPEPTIDE, SOUS FORME DE MICROPARTICULES, ET LEUR PROCEDE DE PREPARATION

(30) Priorität: 10.03.1995 DE 19508612; 11.04.1995 DE 19513659; 17.11.1995 DE 19542837
(43) Veröffentlichungstag der Anmeldung: 07.01.1998
(73) Patentinhaber: Roche Diagnostics GmbH, 68298 Mannheim (DE)
(72) Erfinder: KOLL, Hans, D-82362 Weilheim (DE); WINTER, Gerhard, D-69221 Dossenheim (DE); KISSEL, Thomas, D-35032 Marburg (DE); MORLOCK, Michael, D-68519 Viernheim (DE)
(86) Internationale Anmeldenummer: EP9600980
(87) Internationale Veröffentlichungsnummer: WO9628143

(56) Entgegenhaltungen:
- CHEMICAL ABSTRACTS, vol. 122, no. 12, 20.März 1995 Columbus, Ohio, US; abstract no. 142184, XP002010115 & PROC. INT. SYMP. CONTROLLED RELEASE BIOACT. MATER., 1994, Seiten 286-287, T. KISSEL ET AL.: "RELEASE PROPERTIES OF MACROMOLECULES FROM MICROSPHERES OF A ABA TRIBLOCK COPOLYMER CONSISTING OF POLY (LACTIC-CO-GLYCOLIC ACID) AND POLYOXYETHYLENE."
- CHEMICAL ABSTRACTS, vol. 122, no. 4, 23.Januar 1995 Columbus, Ohio, US; abstract no. 38706, XP002010116 in der Anmeldung erwähnt & J. CONTROLLED RELEASE, Bd. 32, Nr. 2, 1994, Seiten 121-128, L. YOUXIN ET AL.: "IN-VITRO DEGRADATION AND BOVINE SERUM ALBUMIN RELEASE OF THE ABA TRIBLOCK COPOLYMERS CONSISTING OF POLY (L(+)LACTIC ACID),OR POLY (L(+)LACTIC ACID-CO-GLYCOLIC ACID) A-BLOCKS ATTACHED TO CENTRAL POLYOXYETHYLENE B-BLOCKS."
- CHEMICAL ABSTRACTS, vol. 120, no. 10, 7.März 1994 Columbus, Ohio, US; abstract no. 107987, XP002010117 & J. CONTROLLED RELEASE, Bd. 27, Nr. 3, 1993, Seiten 247-257, L. YOUXIN ET AL. : "SYNTHESIS AND PROPERTIES OF BIODEGRADABLE ABA TRIBLOCK COPOLYMERS CONSISTING OF POLY (L-LACTIC ACID) OR POLY (L-LACTIC-CO-GLYCOLIC ACID) A-BLOCKS ATTACHED TO CENTRAL POLY (OXYETHYLENE) B-BLOCKS."

## Beschreibung

Gegenstand der vorliegenden Erfindung sind parenterale pharmazeutische Darreichungsformen in Form von Mikropartikeln (MP) zur kontrollierten Freisetzung von Polypeptiden und Verfahren zur Herstellung dieser Mikropartikel.

Durch das schnelle Fortschreiten der Entwicklung in der Biotechnologie stehen eine Vielzahl von bioaktiven Makromolekülen in ausreichender Menge zur klinischen Anwendung zur Verfügung. Bedingt durch ihre Struktur werden sie im Magen-Darm-Trakt hydrolytisch gespalten und können daher nur parenteral verabreicht werden. Wegen ihrer kurzen Halbwertszeit ist die Entwicklung von parenteralen Depotsystemen sinnvoll, um die Injektionshäufigkeit zu reduzieren und konstante Blutspiegel zu erreichen.

Es werden in der Fach- und Patentliteratur eine Reihe von Depotsystemen, insbesondere mikropartikuläre Systeme, beschrieben, um physiologisch aktive Substanzen nach parenteraler Applikation über einen längeren Zeitraum hinweg möglichst konstant freizusetzen Dabei ist anzumerken, daß Proteine im Vergleich zu niedermolekularen Substanzen aufgrund ihrer komplexen Struktur, ihres hohen Molekulargewichtes und dem - bedingt durch ihre hohe biologische Wirksamkeit - geringen erforderlichen Beladungsgrad einige Besonderheiten aufweisen, die eine erfolgreiche Mikroverkapselung erschweren. So kann je nach Art der eingesetzten Mikroverkapselungsmethode die Proteinstabilität negativ beeinflußt werden und die Freigabe nicht optimal bzw. mit unbefriedigendem Freigabeprofil erfolgen. Das Freigabeverhalten wird einerseits durch das hohe Molekulargewicht und die hydrophile Struktur, andererseits aber auch durch Stabilitätsprobleme (u.a. Aggregation) des Proteins und dem niedrigen Beladungsgrad beeinflußt.

Eine der wichtigsten Herstellungsmethoden für Mikropartikel, wie zum Beispiel Mikrokapseln oder Mikrokugeln, ist das sogenannte "Tripelemulsionsverfahren", das auch bereits zur Mikroverkapselung von Proteinen Anwendung gefunden hat. Grundsätzlich wird bei dieser auch als W/O/W-Technik bezeichneten Methode der Wirkstoff in einer wäßrigen Lösung gelöst bzw. suspendiert, und diese wäßrige Lösung mit einer das Polymer enthaltenden "öligen Lösung" aus einem organischen, nicht mit Wasser mischbaren Lösungsmittel zu einer W/O-Emulsion homogenisiert. Diese W/O-Emulsion wird in eine wäßrige Stabilisator-haltige Lösung (äußere wäßrige Phase) dispergiert, so daß eine Emulsion mit drei Phasen (Tripelemulsion) entsteht. Mittels verschiedener Techniken wird dann die Verdunstung des Lösungsmittels und damit eine Aushärtung der Mikropartikel erreicht. Die gehärteten Mikropartikel werden durch Zentrifugieren und/oder Filtrieren gesammelt und nach Waschen mit Wasser oder geeigneten wässrigen Lösungen und durch Lyophilisation oder Vakuumtrocknung bei Raumtemperatur getrocknet. Als Polymere werden in der Regel Polymere aus Milchsäure (LA = lactid acid) und Glykolsäure (GA = glycolic acid) oder deren Copolymere (PLGA) mit Molekulargewichten zwischen 2.000 und 100.000 und einem Verhältnis von Milchsäure: Glykolsäure zwischen 100:0 bis 50:50 eingesetzt.

Als problematisch kann sich bei der Anwendung des Tripelemulsionsverfahrens der Restlösungsmittelgehalt in den Mikropartikeln erweisen (R. Jalil und J.R. Nixon, J. Microencapsulation 7 (3), 1990, S. 297-325), da das als Polymerlösungsmittel am häufigsten verwendete Dichlormethan aus toxikologischer Sicht bedenklich erscheint. Aber auch aufgrund einer möglichen Beeinflussung der Polymer-Eigenschaften und der Wirkstoffstabilität in der Polymermatrix sollte der Restlösungsmittelgehalt möglichst gering sein.

Die Herstellung von Mikrokapseln mit Hilfe des Tripelemulsionsverfahrens wird z.B. in der europäischen Patentanmeldung EP 0 145 240 (Takeda) offenbart, wobei dort die innere wäßrige Phase eine Viskosität von mindestens 5.000 mPas besitzt bzw. völlig verfestigt ist. Die Erhöhung der Viskosität erfolgt durch Hilfsstoffe wie Gelatine, humanes Serumalbumin, Globulin, Casein, Collagen und Polyaminosäuren. In Anwendungsbeispielen wird die Mikroverkapselung von y-Interferon bzw. Heparin beschrieben.

In der Patentschrift EP 0 190 833 (Takeda) wird das gleiche Herstellungsverfahren beschrieben, nur ist hier die Viskosität der W/O-Emulsion auf einen Wert zwischen 150 - 10.000 mPas einzustellen. Dies geschieht durch Variation der Polymer-Konzentration (PLGA 100/0 - 50/50) und dem Zusatz von natürlichen oder synthetischen hochmolekularen Verbindungen, wie z. B. Proteinen, Kohlenhydraten (Cellulose, Dextrin, Pektin, Stärke, Agar), Polyvinylverbindungen, Polycarbonsäuren oder Polyethylenverbindungen in die wäßrige Phase. Dadurch soll eine stark verminderte Aggregations- und Kohäsionsneigung der Mikropartikel während der Herstellung erreicht werden. In einem Anwendungsbeispiel wird Interferon alpha verkapselt.

In EP 0 442 671 (Takeda) werden bezüglich Aggregationsverhalten, sphärischer Gestalt der Mikropartikel und möglicher Zusätze ähnliche Angaben wie in EP 0 190 833 gemacht. "Arzneistoff-zurückhaltende Substanzen" sind gemäß Patentschrift nicht unbedingt erforderlich. Die in der Beschreibung näher erläuterten und konkret offenbarten Beispiele betreffen das kurzkettige und relativ stabile Peptid TAP144, das ein LHRH-Analogon darstellt.

Auch in der Fachliteratur sind Beispiele für die Mikroverkapselung von Peptiden bzw. Proteinen mit Hilfe der W/O/W-Technik publiziert.

So beschreiben Ogawa et al., (Chem. Pharm. Bull. 1988, Vol. 36, Nr 3, S. 1095-1103) die Mikroverkapselung von Leuprorelinacetat, einem Peptid, unter der Verwendung von PLA (Polymer aus Milchsäure) bzw. PLGA und gehen auch auf das Freisetzungsverhalten des Peptids ein.

Cohen et al., (Pharmaceutical Reseach 1991, Vol. 8, Nr. 6, S. 713) verkapselten FITC-Meerrettich-Peroxidase und FITC-BSA in PLGA-Mikropartikeln mit einem Molekulargewicht von 14.000 oder weniger und einem Anteil Milchsäure/Glykolsäure von 75/25 und fanden sowohl eine Unversehrtheit des Proteins BSA als auch einen Erhalt der Enzym-Aktivität. Jeffery H. et al. (Pharmaceutical Research 1993, Vol. 10, Nr. 3, S. 362) benutzten Ovalbumin als Kernmaterial und konnten die Unversehrtheit des freigesetzten Proteins zeigen. M. S. Hora et al., (Biotechnology 1990, Vol. 8, S. 755) verwendeten Interleukin-2 und modifizierte Formen davon als Kernmaterial und untersuchten das Freisetzungsverhalten von PLGA-Mikropartikeln, die humanes Serumalbumin als Exzipiens enthielten.

Ferner wurden in verschiedenen Publikationen anhand von Modellsubstanzen für Proteine verschiedene Verfahren zur Herstellung von Mikropartikeln auf Basis der PLA- bzw. PLGA-Polymere und der Einfluß von Zuschlagstoffen auf die Proteinstabilität näher untersucht (vgl. W. Lu und G. Park, PDA Journal of Pharmaceutical Science & Technology, 1995, 49: 13 - 19; M.-K. Yeh et al., Journal of Controlled Release, 1995, 33: 437 - 445; M. J. Alonso et al., Vaccine, 1995, 12: 299 - 306; J.P. McGee et al., Journal of Controlled Release, 1995, 34: 77-86). Als Modellproteine wurden hierbei Ovalbumin, Tetanus-toxoid und Carbo-Anhydrase untersucht.

Youxin L. et al. (Journal of Controlled Release 32, (1994) 121-128) beschreiben Depotformen aus ABA-Triblock-Copolymeren (MW:15 000-40 000), deren A-Block ein Copolymer aus Milch- und Glykolsäure ist und deren B-Block eine Polyethylenglykol-Kette (PEG) darstellt. Sie stellten fest, daß diese Mikropartikel das für Aggregation relativ unempfindliche bovine Serumalbumin, das als Modellprotein bei hohem Beladungsgrad (ca. 3-4% w/w) eingesetzt wurde, schnell und kontinuierlich über 2-3 Wochen freisetzen (Polymerzusammensetzung LA:GA:PEG=48:14:38 [Mol%]).

Die im Stand der Technik zur Herstellung von Mikrokapseln bisher häufig verwendeten PLGA-Polymere weisen aufgrund ihres hydrophoben Charakters als entscheidenden Nachteil eine geringe Quellfähigkeit auf, wodurch der Wassereintritt in das Innere der Depotform nur langsam erfolgen kann. Dadurch ist eine Diffusion der Proteinmoleküle durch die Polymerschichten nur erschwert möglich, was eine unbefriedigende Freisetzungsrate zur Folge hat. Dies ist insbesondere beim Einschluß sehr geringer Polypeptidmengen, d.h. bei niedrigem Beladungsgrad, in die Mikropartikel der Fall. Außerdem bewirkt die langsame Wasseraufnahme wegen der geringen verfügbaren Wassermenge eine hohe lokale Proteinkonzentration, wodurch eine Bildung von hochmolekularen Proteinaggregaten gefördert wird. Diese können wiederum wegen ihres hohen Molekulargewichtes nicht mehr freigesetzt werden. Eine therapeutisch zuverlässige Dosierung des Wirkstoffes ist dann nicht mehr gewährleistet. Ferner kann es bedingt durch den hohen Anteil der Proteinaggregate zu unerwünschten immunologischen Reaktionen kommen. Nur sehr stabile Proteine mit relativ hohen Beladungsgraden von beispielsweise mehr als 5 % können mit akzeptabler Rate und ohne Bildung von Aggregaten über einen längeren Zeitraum hinweg freigesetzt werden.

Es zeigte sich ferner, daß auch hydrophile ABA-Triblock-Copolymere eine kontinuierliche Freisetzung von Polypeptiden über einen Zeitraum von zwei Wochen dann nicht gewährleisten können, wenn der Polypeptidgehalt in den Mikropartikeln sehr gering ist, d.h. wenn der Beladungsgrad niedrig ist. Ein niedriger Beladungsgrad liegt dann vor, wenn nur geringe Polypeptidmengen im Polymer eingeschlossen sind. Ein ähnliches nachteiliges Verhalten in der Freisetzung ist festzustellen, wenn aggregationsempfindliche Polypeptide verwendet werden. In diesen Fällen werden auch mit dem hydrophilen ABA-Triblock-Copolymer eine verstärkte Aggregatbildung und inakzeptable Freisetzungszeiträume von weniger als zwei Wochen beobachtet. Dies führt insgesamt zu unbefriedigenden Freigaberaten des Wirkstoffes aus dem Polymer.

Aufgabe der Erfindung war es, Polypeptid-enthaltende Mikropartikel herzustellen, in denen die Aggregation des Wirkstoffes auch für aggregationsempfindliche Polypeptide möglichst gering gehalten bzw weitgehend vermieden werden soll, und so das Polypeptid in einer möglichst unversehrten Form in den Mikropartikeln enthalten ist. Die Mikropartikel sollen eine kontinuierliche Freisetzung der Polypeptide über einen Zeitraum von mindestens zwei Wochen gewährleisten. Dies sollte vor allem bei solchen Mikropartikeln erzielt werden, die einen niedrigen Beladungsgrad an Wirkstoff aufweisen. Insbesondere sollten diese Freisetzungszeiträume für geringe Polypeptidmengen von bis zu etw 3 % (bezogen auf die Gesamtmikropartikelmenge) gelten.

Daneben war es Aufgabe der Erfindung, ein Mikroverkapselungsverfahren bereitzustellen, mit dem diese gewünschten Mikropartikel herstellbar sind und das einen toxikologisch unbedenklichen Restlösungsmittelgehalt in den Mikropartikeln gewährleistet.

Die Aufgabe der Erfindung wird gelöst durch Mikropartikel, die aus einer bioabbaubaren Polymermatrix bestehen, in die das Polypeptid eingebettet ist, wobei als Polymer ein ABA-Triblock-Copolymer verwendet wird, dessen A-Block ein Copolymer aus Milch- und Glykolsäure ist und dessen B-Block eine Polyethylenglykol-Kette darstellt, und die Zuschlagstoffe enthalten, die ausgewählt sind aus der Gruppe Serumproteine, Polyaminosäuren, Cyclodextrine; Cyclodextrinderivate; Saccharide, wie z. B. Di- und Polysaccharide; Aminozucker; Aminosäuren; Detergenzien; organische Carbonsäuren sowie Gemische dieser Zuschlagstoffe.

Als Saccharide kommen beispielsweise die Disaccharide Trehalose, Saccharose, Maltose, in Frage. Polysaccharide sind beispielsweise Raffinose, Stärke, Maltodextrine, Alginate oder Dextran. Ein geeigneter Aminozucker ist beispielsweise das Chitosan. Ein bevorzugtes Cyclodextrinderivat im Sinne der Erfindung ist beispielsweise das Beta-Hydroxypropyl-Cyclodextrin (HPCD). Als Serumproteine kommen insbesondere Humanserumalbumin und bovines Serumalbumin in Frage.

Als organische Carbonsäuren kommen aliphatische und cyclische Monocarbonsäuren in Frage, beispielsweise Benzoesäure, Essigsäure, Propionsäure, Buttersäure, Isobuttersäure, Valeriansäure, Acrylsäure, Crotonsäure sowie deren durch Hydroxygruppen substituierten Derivate, wie z.B. p-Hydroxybenzoesäure, β-Hydroxybuttersäure, Salicylsäure, Milchsäure oder Glykolsäure. Insbesondere eignet sich Benzoesäure. Die Carbonsäuren werden im Rahmen der Herstellung der Mikropartikel im wesentlichen der organischen Phase (Polymerphase) zugesetzt, in der das Polymer gelöst oder suspendiert ist. Die zugesetzte Menge an Carbonsäuren bewegt sich im Bereich von bis zu 30 Gew.-%, bezogen auf die Menge der fertigen Mikropartikel, vorzugsweise bis zu 20 Gew.-%, insbesondere 1 - 15 Gew.-%. Die Verwendung von Monocarbonsäuren, wie z.B. Benzoesäure, als Zuschlagstoff bewirkt überraschenderweise eine Verbesserung der Freisetzung der Polypeptide aus den Mikropartikeln. Ein durch den Zusatz von Carbonsäuren zu erwartender beschleunigter Polymerabbau konnte dabei im Falle der ABA-Triblock-Copolymere nicht festgestellt werden.

Vorteilhaft im Sinne der Erfindung sind auch Gemische der zuvor genannten Zuschlagstoffe. Beispielhaft seien erwähnt Gemische aus Dextranen und Polyaminosäuren. So sind z.B. Gemische aus Dextran und Poly-L-Arginin oder Dextran und Poly-L-Histidin besonders vorteilhaft in Bezug auf die Erniedrigung der Aggregatbildung des Polypeptides im Mikropartikel. Bevorzugt als Zuschlagstoffe sind auch Gemische aus Cyclodextrinen oder Cyclodextrinderivaten mit Aminosäuren oder Polyaminosäuren. Auch Detergenzien und Triglceride wie beispielsweise Tween 20® , Tween 80® , Pluronic® oder Miglyol® sind als Zuschlagstoffe im Sinne der Erfindung geeignet.

Als Polyaminosäuren kommen sowohl die entsprechenden (D) oder (L) bzw. Poly-(D,L)-aminosäuren in Frage. Besonders bevorzugt ist Polyarginin mit einem Molekulargewicht von 5.000 - 150.000, insbesondere 5.000 bis 50.000, sowie Polyhistidin mit einem Molekulargewicht von 5.000 - 50.000, insbesondere 15.000 - 50.000.

Mit den genannten erfindungsgemäßen Zuschlagstoffen ist es möglich, den Gesamt-Aggregat-Anteil des Polypeptides unter 5% zu senken.

Als Polypeptide kommen im Sinne der Erfindung physiologisch aktive Polypeptide mit einem Molekulargewicht zwischen 2.000 bis 200.000 D in Frage. Vorzugsweise beträgt das Molekulargewicht mindestens 5.000, 10.000 oder 20.000 D. Insbesondere kommen Polypeptide mit einem Molkulargewicht von bis zu 100.000, vorzugsweise bis zu 50.000 Dalton in Frage. Solche Polypeptide sind insbesondere biologisch aktive Makromoleküle, deren Muteine, Analoga, sowie Deletions- oder Substitutionsvarianten, die zu therapeutischen Zwecken eingesetzt werden können. Folgende Polypeptide werden beispielhaft erwähnt: Erythropoietin (EPO), Parathormon (PTH), G-CSF, TNF, NGF oder EGF, sowie deren durch Deletionen oder Substitutionen in der Aminosäurekette ableitbaren Derivate. Weitere Polypeptide sind: Interferone (α, β, y- Interferon), Kolonie stimulierende Faktoren, Interleukine, Makrophagen aktivierende Faktoren, B-Zell-Faktoren, T-Zell-Faktoren, Immunotoxine, Lymphotoxine, TGF, Thrombopoietin (TPO), Renin-Inhibitoren, Collagenase-Inhbitoren, EGF, Wachstumshormone, PDGF, Knochenwachstumsfaktoren, BMP (bone morphogenic proteins), Insulin, IGF-BP (insulin-like growth factor binding proteins), ANP (atrial natriuretic peptides), Calcitonin, FSH, LH, NGF, Glukagon, TSH (thyroid stimulating hormone), monoklonale oder polyklonale Antikörper. Besonders geeignete Polypeptide sind im Sinne er vorliegenden Erfindung aggregationsempfindliche Polypeptide, wie beispielsweise EPO.

Der Polypeptidgehalt in den Mikropartikeln beträgt zwischen 0,01 bis 5 Gew.-%, bezogen auf die Gesamtmikropartikelmenge. Bevorzugt beträgt der Beladungsgrad 0,1 - 3 Gew.-%, insbesondere 0,1 - 2 Gew.-%, und bevorzugt 0,1 - 1 Gew.-%. Insbesondere können Mikropartikel mit einem sehr geringen Beladungsgrad von bis zu 1 Gew.-% hergestellt werden. Für aggregationsempfindliche Proteine beträgt der bevorzugte Beladungsgrad 0,1 - 1%, insbesondere 0,2 - 0,6 %. Als untere Grenze kommt ein Beladungsgrad von etwa 0,01; 0,05 bzw. 0,1 Gew.-% in Frage. Die Menge des in den Mikropartikeln enthaltenen Wirkstoffes ist abhängig von der in jedem Einzelfall zu bestimmenden Dosierung und der therapeutischen Breite des jeweiligen Wirkstoffes. Im Falle von EPO beträgt die Menge an Wirkstoff etwa 10 µg - 100 µg pro 10 mg Mikropartikelmenge. Insbesondere werden etwa 10 - 70 µg, bevorzugt 30 - 50 µg eingesetzt. Bei einer spezifischen Aktivität von EPO von etwa 160.000 U/mg entspricht dies einer Dosierung von 1.600 - 16.000 U pro 10 mg Mikropartikelmenge (10 - 100 µg pro 10 mg Mikropartikelmenge). Bevorzugt wird die zu verabreichende Mikropartikelmenge an der gewünschten Dosierung von EPO (in U) festgelegt. Wenn beispielsweise der Beladungsgrad 0,4 % beträgt (entspricht 40 µg EPO pro 10 mg Mikropartikel) und die Dosierung von EPO 20.000 U (entspricht 125 µg EPO) betragen soll, ist eine Mikropartikelmenge von 31,25 mg zu verabreichen. Diese Menge entspricht einer voraussichtlichen Monatsdosis von EPO im DDS-System

Überraschenderweise wurde gefunden, daß die Verwendung von ABA-Triblock-Copolymeren in Kombination mit Zuschlagstoffen eine kontinuierliche Freigabe der Polypeptide über einen längeren Zeitraum hinweg - mindestens jedoch zwei Wochenermöglicht, und durch die Zuschlagstoffe ein erheblicher aggregationsmindernder Effekt erreicht wird. Erfindungsgemäß kommen ABA-Blockpolymere in Frage, deren A-Block ein Molekulargewicht zwischen 2.000 und 150.000 besitzt, und deren B-Block ein Molekulargewicht zwischen 1.000 und 15.000 besitzt. Insbesondere weist der B-Block ein Molekulargewicht zwischen 3.000 bis 10.000 auf. Besonders bevorzugt sind ABA-Blockpolymere mit einem Molekulargewicht von 5.000 - 50.000 Dalton, vorzugsweise 10.000 - 30.000, und einer Polydispersität von 1,1 - 8,5 oder 1,1 - 5,5, vorzugsweise 1,5 - 4,5 und insbesondere bevorzugt von 2 - 4.

Tabelle 5 gibt eine Übersicht über die erfindungsgemäß verwendbaren ABA-Copolymere, die sich in ihrer Zusammensetzung bezüglich des Laktid/Glykolid/ PEG-Anteils, dem Molekulargewicht und der Polydispersität unterscheiden. Erfindungsgemäß beträgt der Polyethylenglykolanteil (PEG-Anteil) im Blockpolymer 20 bis 50 Mol-%, bezogen auf die Gesamtpolymermenge, vorzugsweise 25 bis 45 Mol-%. Als besonders vorteilhaft für die Freigabedauer und für die kontinuierliche Freisetzung des Wirkstoffes hat sich ein PEG-Anteil von 30 bis 40 Mol-%, insbesondere 30 bis 38 Mol-%, bevorzugt 30 bis 35 Mol-% erwiesen. Bevorzugt beträgt der prozentuale Gehalt von PEG im ABA-Blockcopolymer etwa 32 oder 33 Mol-%.

Der prozentuale Gehalt von LA im ABA-Blockcopolymer beträgt vorzugsweise 40 bis 60 Mol-%, insbesondere 45 - 60 Mol-%. Bevorzugt sind Molprozente von etwa 46 %, 51 % oder 57 %. Der prozentuale Gehalt von GA im ABA-Blockcopolymer beträgt vorzugsweise 5 bis 25 %, insbesondere 10 bis 25 %. Bevorzugte Prozentangaben sind etwa 11 %, 16 % oder 22 %.

Das Verhältnis von Milchsäure zu Glykolsäure im Blockpolymer liegt zwischen 1:1 bis 5:1, insbesondere beträgt es zwischen 1,5:1 bis 4,5:1. Besonders bevorzugt ist ein Verhältnis LA/GA von etwa 2:1 bis 4:1. Erfindungsgemäß besonders bevorzugte ABA-Triblock-Copolymere sind Polymere mit einem Verhältnisanteil von LA/GA = 4 : 1 und einem Polyethylenglykolgehalt von 30-38 %. Insbesondere kommt ein Polymer mit einem Verhältnisanteil LA:GA:PEG = 57:11:32; 51:16:33; 50:12:38 oder 46:22:32 in Frage.

Die letztgenannten Polymermodifikationen bieten ein Optimum an Abbaugeschwindigkeit und PEG-Gehalt. Ein höherer PEG-Gehalt führt zwar zu einem noch schnelleren Abbau, andererseits aber auch zu einer Verschlechterung der mechanischen Eigenschaften der Mikropartikel und möglicherweise auch zu Wechselwirkungen zwischen PEG und Polypeptid.

Die Herstellung der ABA-Triblock-Copolymere kann nach literaturbekannten Verfahren (s. Journal of Controlled Release 27, 1993, 247-257) durchgeführt werden.

Überraschend wurde gefunden, daß die erfindungsgemäßen Zuschlagstoffe neben ihrem aggregationsmindernden Effekt eine signifikante Erhöhung der Freisetzungsdauer bewirken können, verglichen mit ABA-Mikropartikeln ohne Zuschlagstoffe. Dies gilt insbesondere für die erfindungsgemäßen Serumproteine, die eine Erhöhung der Freisetzungsdauer des Polypeptids auf beispielsweise bis zu 29 Tagen hervorrufen (vgl. Beispiel 4). Als Serumproteine werden bevorzugt bovines oder humanes Serumalbumin eingesetzt. Werden die erfindungsgemäßen Zuschlagstoffe, insbesondere BSA und Beta-Hydroxypropyl-Cyclodextrin, PLGA-Mikropartikeln zugesetzt, so tritt ebenfalls ein aggregationsmindernder Effekt und somit eine Stabilisierung der aggregationsempfindlichen Polypeptide ein.

Derartig lange Freisetzungszeiträume von bis zu 29 Tagen sind von Polypeptiden aus Mikropartikeln auf Basis von PLGA oder ABA-Triblock-Copolymeren bislang nur bei hohem Beladungsgrad bekannt, jedoch nicht für solche Fälle, in denen die Wirkstoffmenge in den Mikropartikeln sehr gering ist, wie beispielsweise im Fall von EPO. Wird EPO in einem höheren Beladungsgrad (z.B. ca. 3 %) in den ABA-Mikropartikeln eingeschlossen, wird das Protein über 29 Tage freigesetzt (s. Tab. 4 B). Insbesondere konnte festgestellt werden, daß eine verlängerte Freisetzung erzielt wird, wenn eine Monocarbonsäure, insbesondere Benzoesäure, der Polymerphase bei der Herstellung der Mikropartikel zugesetzt wird. Dies gilt insbesondere im Fall der zuvor genannten niederen Beladungsgraden.

Die erfindungsgemäßen Mikropartikel können als Zuschlagstoffe auch Aminosäuren wie z.B. Arginin, Glycin, Lysin oder Phenylalanin, Cyclodextrine oder Cyclodextrinderivate wie z.B. Beta-Hydroxypropyl-Cyclodextrin (HPCD) enthalten. Ebenso können erfindungsgemäß als Zuschlagstoffe Polyaminosäuren wie z.B. Polyarginin oder Polyhistidin eingesetzt werden oder auch Gemische aus Cyclodextrinen oder Cyclodextrinderivaten mit Aminosäuren oder Polyaminosäuren wie z.B. HPCD mit Polyarginin. Auch Gemische aus Dextranen mit Cyclodextrinderivaten, z.B. mit HPCD, oder mit Cyclodextrinen finden Verwendung. Die verwendeten Dextrane besitzen ein Molekulargewicht zwischen 20.000 und 60.000, besonders bevorzugt ist Dextran 40.000.

Neben den Serumproteinen werden erfindungsgemäß als Zuschlagstoffe Gemische aus Dextranen und Polyarginin oder Gemische aus Dextranen und Polyhistidin besonders bevorzugt eingesetzt.

Die erfindungsgemäßen Mikropartikel enthalten die Zuschlagstoffe in einer Menge von 0,5 - 40 Gew.-%, bezogen auf die Gesamtmikropartikelmenge, vorzugsweise von 1 - 30%. Insbesondere bevorzugt sind 1 - 20 Gew.-%. Im Fall der Saccharide werden vorzugsweise Mengen von 5 - 15 Gew.-% eingesetzt. Im Fall der Polyaminosäuren beträgt die Menge der Zuschlagstoffe insbesondere 1 - 5 Gew.-%. Cyclodextrin und Cyclodextrinderivate werden vorzugsweise in einer Menge von 2 - 20 Gew.-% zugesetzt. Die Menge an BSA oder HSA beträgt bevorzugt 2 - 20 Gew.-% bezogen auf das Gesamtpartikelgewicht. Die Menge an Carbonsäuren beträgt insbesondere bis zu 15 %, bevorzugt etwa 10 %.

Die Erfindung betrifft auch ein Verfahren zur Herstellung von Polypeptid enthaltenden Mikropartikeln mit Hilfe des Tripelemulsionsverfahrens, das dadurch gekennzeichnet ist, daß das bei der Herstellung der "öligen" bzw. organischen Phase durch Lösen eines Polymers in einem organischen, nicht wassermischbaren Lösungsmittel als Polymer ein ABA-Triblock-Copolymer eingesetzt wird, dessen A-Block ein Copolymer aus Milch- und Glykolsäure ist und dessen B-Block eine Polyethylenglykol-Kette darstellt und dem wäßrig gelösten Polypeptid, das in der organischen Phase emulgiert wird, Zuschlagstoffe zugesetzt werden, die ausgewählt sind aus der Gruppe Serumproteine, Polyaminosäuren, Cyclodextrine; Cyclodextrinderivate; Saccharide, wie z. B. Di- und Polysaccharide; Aminozucker; Aminosäuren; Detergenzien oder Carbonssäuren sowie deren Gemische.

Es hat sich gezeigt, daß der erste Homogenisierungsschritt (Bildung der W/O-Emulsion) offensichtlich für die Bildung von Polypeptid-Aggregaten besonders verantwortlich zu sein scheint. Erfindungsgemäß wurde deshalb die Dispergierzeit von 60 auf 30 Sekunden verkürzt, als Homogenisator ein Ultra-Turrax eingesetzt und das Gewichtsverhältnis Wasser/organische Phase (bevorzugt Dichlormethan) von 5 auf bis zu 20 % (Gewichtsprozent) erhöht. Vorzugsweise wird im ersten Homogenisierungsschritt zweimal etwa 30 Sekunden, mit einer Pause von etwa 30 Sekunden, dispergiert. Besonders vorteilhaft wird einmal etwa 30 Sekunden lang dispergiert. Durch diese Modifizierung der Herstellungsbedingungen konnte eine leichte Abnahme des Aggregatanteils erreicht werden.

Im Sinne des erfindungsgemäßen Herstellverfahrens ist es besonders vorteilhaft, wenn während des Herstellungsprozeßes alle Lösungen und Geräte während der gesamten Herstelldauer auf 0 - 6 °C gekühlt werden. Hierdurch wird eine besonders günstige Aggregatreduktion des Proteins erzielt. Es ist in diesem Fall sogar möglich, auf den Zusatz von aggregationshemmenden Zuschlagstoffe weitgehend zu verzichten. Im Vergleich zur Herstellung der Mikopartikel bei Raumtemperatur konnte auf diese Weise eine deutliche Reduzierung des Aggregatgehaltes der verwendeten Polypeptide in den Mikropartikeln erreicht werden (Reduktion von 10 - 20 % Aggregatgehalt auf einen Aggregatgehalt von 2 - 5 %).

Das Gewichtsverhältnis Wasser/organische Phase von 20-25 % (3 -4 Teile organisches Lösungsmittel/ein Teil Wasser) ermöglicht außerdem das Einbringen einer größeren Menge von Zuschlagstoffen in die innere wäßrige Phase.

Die erfindungsgemäßen Mikropartikel besitzen überraschenderweise auch einen äußerst geringen Restlösungsmittelgehalt. Die mit ABA-Polymer hergestellten Mikropartikel enthalten weniger als 1 %, vorzugsweise weniger als 0,1 %, insbesondere weniger als 0,01 % Restlösungsmittel, wie z.B. Dichlormethan. Offensichtlich wurde durch die gewählten Prozeßparameter eine nahezu vollständige Entfernung des Dichlormethans aus den entstehenden Mikropartikeln erreicht, was vor allem auf das günstige Volumen-Verhältnis der organischen zur äußeren wäßrigen Phase zurückzuführen ist.

Um den Einfluß von Restwasser auf die Proteinstabilität auszuschließen, wurde auch der Restwassergehalt in den Mikropartikeln bestimmt. Der ermittelte Wassergehalt von 0,2% zeigte, daß der Wasseranteil der eingesetzten inneren wässrigen Phase fast vollständig entfernt werden konnte.

Untersuchungen zur Lagerstabilität der erfindungsgemäßen Mikropartikel haben gezeigt, daß diese mindestens 2 Monate bei Raumtemperatur (20-25°C) lagerstabil sind und keine Veränderungen hinsichtlich der Aggregat-Bildung und dem Freisetzungsverhalten auftreten.

Gegenstand der Erfindung ist weiterhin die Verwendung von pharmazeutischen Zuschlagstoffen ausgewählt aus der Gruppe Serumproteine, Polyaminosäuren; Cyclodextrine; Cyclodextrinderivate; Saccharide; Aminozucker; Aminosäuren; Detergenzien sowie Gemische dieser Zuschlagstoffe zur Vermeidung der Aggregatbildung von aggregationsempfindlichen Polypeptiden bei der Herstellung von Polypeptid-enthaltenden Mikropartikeln.

In den folgenden Ausführungsbeispielen wird die Erfindung näher erläutert, ohne sie darauf zu beschränken.

### Beispiel 1:

### Verfahren zur Herstellung von Mikropartikeln enthaltend EPO (W/O/W-Methode)

Ein D,L-PLGA-Polymer (LA:GA=50:50; RG503) wurde von Boehringer Ingelheim bezogen und ein ABA-Copolymer (LA:GA:PEG=50:12:38) wurde wie in "Journal of Controlled Release 27, 1993, 247-257" beschrieben, hergestellt.

Es wurde je eine Lösung aus dem D,L-PLGA- und ABA-Block-Polymer in Dichlormethan hergestellt, indem 700 mg des Polymers in 2,5 ml (3,3 g) Dichlormethan gelöst wurden. 3,5 mg EPO (in 0,2 ml Natrium-Phosphat-Puffer, pH 7,4) werden je nach Bedarf mit Zuschlagstoffen (1 % - 20 Gew.% bezogen auf die Gesamtmikropartikelmenge) versetzt und mit Wasser auf 0,8 ml (0,8 g) Endvolumen aufgefüllt.

Die wäßrige EPO-Lösung wird zur Polymerlösung gegeben und mit Hilfe eines Ultra-Turrax (30 Sekunden, 30s Pause, erneut 30s, 20-24°C, 20.000 U bzw. einmal für 30 Sekunden) eine W/O-Emulsion hergestellt. Anschließend wird die W/O-Emulsion durch Einspritzen in 300 ml wäßrige 0,1 % PVA-Lösung mit Hilfe eines Ultra-Turrax bei 8000 U/min für 30 Sekunden dispergiert (Herstellung einer W/O/W-Tripelemulsion).

Die W/O/W-Emulsion wird zur Verdunstung der organischen Dichlormethanphase für 2 bis 3 Stunden bei Raumtemperatur mit Hilfe eines Flügelrührers gerührt (solvent evaporation). Die gebildeten und gehärteten MP werden durch Abnutschen isoliert, zweimal mit je 200 ml Wasser gewaschen und lyophilisiert. Die Mikropartikel werden in einem Exsikkator über Blaugel bei 4 °C - 8°C gelagert.

### Beispiel 2:

### Stabilisierung von EPO-haltigen Mikropartikeln

Es wurden nach herkömmlichen Methoden, wie in Beispiel 1 beschrieben, Mikropartikel hergestellt, wobei unterschiedliche Zuschlagstoffe in unterschiedlichen Mengen, bezogen auf die Gesamtmikropartikelmenge, eingesetzt wurden.

Die Aggregatbildung des Wirkstoffes EPO wurde anschließend mittels SDS-PAGE nach der Extraktion von EPO aus den Mikropartikeln (a) oder der Solvatation der Mikropartikel in DMSO oder DMSO/DMF-Gemisch (30:70) (b) folgendermaßen bestimmt:
a) 10 mg MP wurden in 300 µl CH₂Cl₂ gelöst und EPO durch Zugabe von 700 µl Aceton ausgefällt. Das EPO-Präzipitat wurde abzentrifugiert, 2x mit 1 ml CH₂Cl₂/Aceton-Gemisch (1:3) gewaschen und anschließend in der speed vac getrocknet. Der Niederschlag wurde in Probenpuffer für SDS-PAGE (Zusammensetzung: 60 mM Tris-HCl, pH 6,8; 2 % SDS; 10 % Glycerin; 0,001 % Bromphenolblau) gelöst, auf ein 12,5 oder 15%-iges SDS-Gel geladen und einer Elekrophorese unterzogen.
b) 10 mg MP wurden in 200 µl DMSO/DMF (30:70) gelöst. 25 µl (entspricht ca. 5 µg EPO) wurden direkt auf ein 15 %-iges SDS-Gel geladen und einer Elektrophorese unterzogen.
   Nach Abschluß der Elektrophorese wurden die Gele entweder:
   aa) mit Coomassie angefärbt und mittels eines Laserscanners densitometrisch vermessen, oder
   bb) auf Nitrocellulose geblottet, mittels eines EPO-spezifischen Antikörpers die EPO-haltigen Banden angefärbt und mittels eines Laserscanners densitometrisch vermessen.

Es zeigte sich, daß in ABA-Mikropartikeln (LA:GA:PEG = 50:12:38) der EPO-Gesamt-Aggregat-Anteil von ca. 15-30% durch den Einschluß von BSA auf unter 1% verringert werden konnte. Weiterhin zeigten Poly-L-Arginin bzw. Poly-L-Histidin, auch in Kombination mit Dextran 40.000 eine deutliche Aggregat-reduzierende Wirkung. Diese Zuschlagstoffe wurden in einer Menge von 1 bis 10 Gew.-%. bezogen auf die Gesamtmikropartikelmenge, eingesetzt (vgl. Tabelle 1).

Auch in EPO-PLGA-Mikropartikeln wird durch Zuschlagstoffe eine Aggregat-Reduzierung erreicht. Hier erwiesen sich insbesondere BSA und β-Hydroxy-Propyl-Cyclodextrin als äußerst wirkungsvoll (Aggregatanteil unter 1%) (vgl. Tabelle 2). In den Mikropartikeln mit PEG als Zuschlagstoff wurde dagegen ein Anstieg des Aggregatanteils festgestellt. Daneben wurde bei PEG bzw. Pluronic F127 haltigen Mikropartikeln mit mindestens 4% Hilfsstoffanteil ein vermehrtes Auftreten deformierter Mikropartikel beobachtet.

**Tabelle 1:**

| Zuschlagstoffe und ihr Einfluß auf die Aggregat-Situation | | |
|---|---|---|
| Zuschlagstoff | % ww vom Gesamtpartikel | Aggregation ↑ = erhöht - = unverändert ↓ = erniedrigt |
| ohne (Raumtemperatur) | | 10 - 20 % |
| ohne (0 - 4 °C) | | ↓ |
| bovines Serumalbumin | 5 | ↓↓↓ |
| bovines Serumalbumin | 10 | ↓↓↓ |
| Dextran 40.000 | 5 | ↓ |
| Dextran 20.000 | 5 | - |
| Poly-L-Arginin | 1-5 | ↓↓ |
| Poly-L-Histidin | 1-5 | ↓↓ |
| Poly-L-Arginin β-Hydroxypropyl-Cyclodextrin | 2,5 2,5 | ↓ |
| Dextran 40.000 β-Hydroxypropyl-Cyclodextrin | 2,5 2,5 | ↓↓ |
| Poly-L-Arginin Dextran 40.000 | 1-5 5 | ↓↓ |
| Poly-L-Histidin Dextran 40.000 | 1-5 5 | ↓↓ |
| β-Hydroxypropyl-Cyclodextrin | 5-15 | ↓ |
| Arginin | 5 | ↓ |
| Benzoesäure | 10 | - |
| Tween 20 | 0,5 | ↓ |
| Pluronic F68 | 0,5 | ↓ |
| Pluronic F127 | 0,5 | ↓ |
| Zum Vergleich: | Δ | ↑↑ |
| 100 mM Na-Phosphat | 15 | |

**Tabelle 2:**

| Zuschlagstoffe und ihr Einfluß auf die Aggregat-Situation in PLGA-Mikropartikeln | | | |
|---|---|---|---|
| Zuschlagstoff | % ww vom Gesamtpartikel | Aggregation ↑ = erhöht - = unverändert ↓ = erniedrigt | initialer Burst [%] |
| ohne | | 8 - 10 % | 29 |
| BSA | 5 | ↓↓ | 40 |
| BSA | 10 | ↓↓ | 40 |
| CD | 5 | ↓↓ | 30 |
| CD | 10 | ↓↓ | 40 |
| CD | 15 | ↓↓ | 40 |
| CD/PEG 10.000 | 5/5 | ↓ | 7 |
| CD/Pluronic F127 | 5/5 | ↓ | 8 |
| CD/Trehalose | 5/5 | ↓ | 30 |
| Dextran 40.000 | 5 | ↓ | 10 |
| D40/Poly-(L)-Arginin | 5/1 | ↓ | n.b. |
| Arginin | 0,2 | ↓ | 15 |
| Arginin | 4,8 | ↓ | 18 |
| PEG 1.550 | 0,43 | - | 12 |
| PEG 1.550 | 4,3 | ↑ | 1.3 |
| PEG 10.000 | 10 | ↑ | 0.6 |
| PEG 35.000 | 10 | ↑ | n.b. |
| Pluronic F127' | 10 | ↓ | 20 |
| n.b. : nicht bestimmt | | | |

### Beispiel 3:

### Einfluß von Zuschlagstoffen auf die Freisetzung von EPO aus PLGA-Mikropartikeln

Es wurden Mikropartikel auf Basis von D,L-PLGA-Polymeren (RG503, MG= 40 000; LA:GA= 50:50; Polydispersität 2,4) mit einem Wirkstoffgehalt von EPO von 0,5 % (bezogen auf die Gesamtmikropartikelmenge) gemäß Beispiel 1 hergestellt, wobei bei der Herstellung unterschiedliche Zuschlagstoffe (% w/w bezogen auf die Gesamtmikropartikelmenge) eingesetzt wurden.

Die Bestimmung der Freisetzungsrate (in % der verkapselten Wirkstoffmenge/pro Tag) erfolgte folgendermaßen:
Jeweils 15 mg Mikropartikel wurden in 2 ml Eppendorfgefäßen eingewogen und mit 1,5 ml PBS-Puffer und 0,01 % Tween 20, pH 7,4 versetzt. Diese Röhrchen wurden in einem auf 37 °C thermostatierten, rotierenden Metallblock (Rotatherm, Fa. Liebisch, 30 U/Min.) gestellt. Nach den vorgegebenen Probenahmezeiten wurden die Proben gezogen und das verbleibende Freigabemedium jeweils vollständig durch neues Medium ausgetauscht. Es wurden die Freisetzungsraten für folgende Mikropartikel bestimmt: 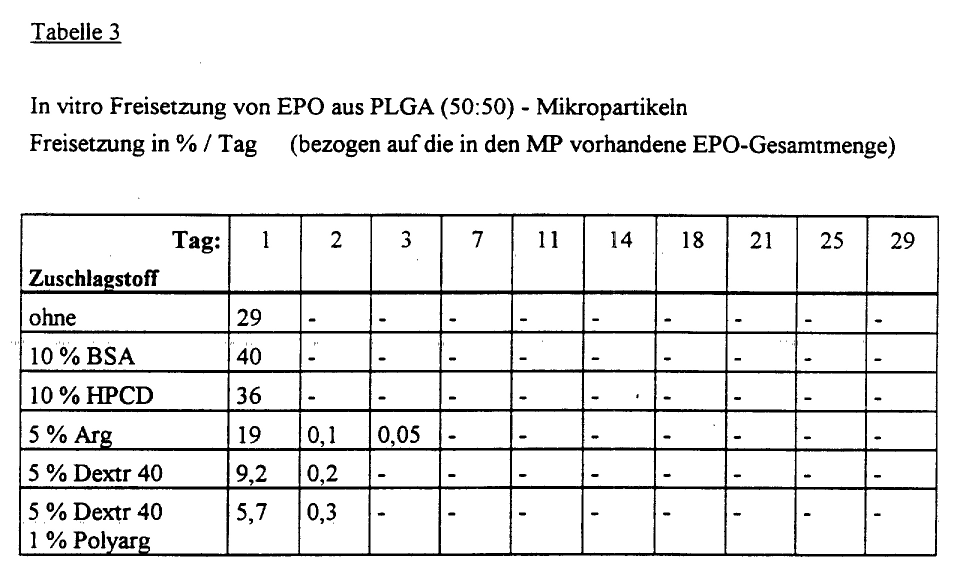 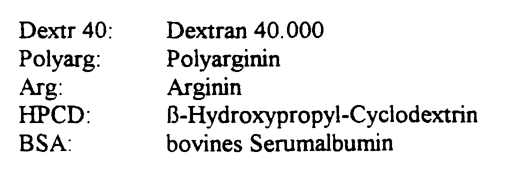
Aus diesen Ergebnissen wird deutlich, daß in PLGA-Mikropartikeln unabhängig von den Zusatzstoffen die EPO-Freisetzung nur maximal 24-36 Stunden andauert und dann keine weitere kontinuierliche Freisetzung erfolgt. Durch die Zuschlagstoffe wird lediglich die Höhe des initialen Burst verändert. Eine protahierte Freigabe des EPO konnte mit den Zuschlagstoffen nicht erreicht werden.

### Beispiel 4:

### Einfluß von Zuschlagstoffen auf die Freisetzung von EPO aus ABA-Mikropartikeln

Es wurden Mikropartikel auf Basis von ABA-Triblock-Copolymeren mit LA:GA:PEG=57:11:32 (Polymer A) und LA:GA:PEG=50:12:38 (Poymer B) mit einem Wirkstoffgehalt von EPO von jeweils 0,5 Gew.-% (bezogen auf die Gesamtmikropartikelmenge) gemäß Beispiel 1 hergestellt, wobei unterschiedliche Zuschlagstoffe (% w/w bezogen auf die Gesamtmikropartikelmenge) eingesetzt wurden. Die Bestimmung der Freisetzungsraten wurde wie in Beispiel 3 beschrieben durchgeführt. Die Ergebnisse sind in Tabelle 4 A zusammengefaßt. Die Polymere A und B waren hinsichtlich des Freisetzungsverhaltens des Wirkstoffes qualitativ weitgehend vergleichbar.

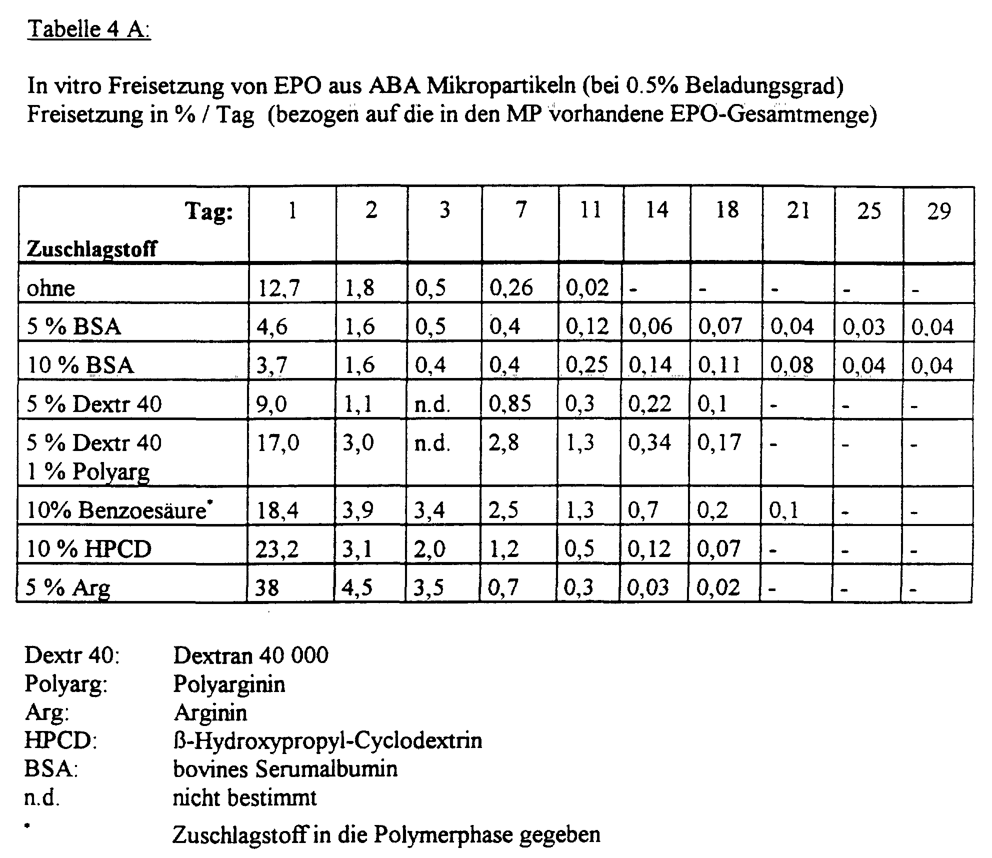

Aus Tabelle 4 A wird deutlich, daß im Gegensatz zu den PLGA-Mikropartikeln bei den ABA-Mikropartikeln eine kontinuierliche Freisetzung von EPO bis beispielsweise zum 29. Tag erzielt werden kann, insbesondere bei der Verwendung von BSA als Zuschlagsstoff.

Vergleicht man die in vitro Freisetzung von EPO aus PLGA (50:50) - Mikropartikeln und ABA-Mikropartikeln verschiedener Monomerzusammensetzungen mit einem Wirkstoffgehalt von 0,5 % bzw. 3,4 % - jeweils ohne Zusatzstoffe - so wird die Überlegenheit der erfindungsgemäßen ABA-Mikropartikel deutlich:

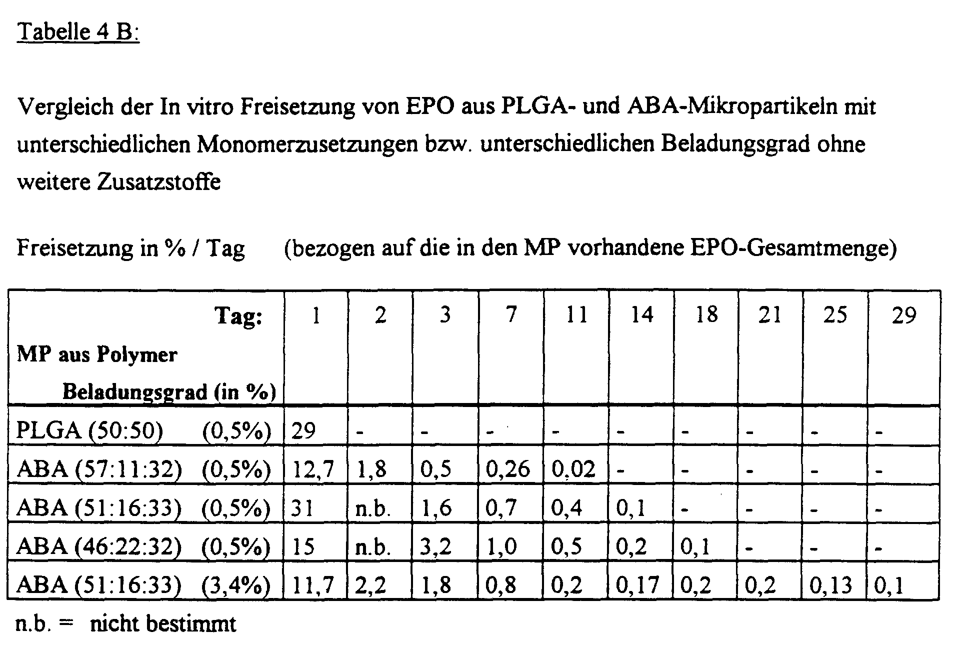

Aus der Tabelle 4 B ist zu entnehmen, daß bei geringem Beladungsgrad (0,5 %) die Freisetzung aus ABA-Mikropartikeln auf 11-18 Tage begrenzt ist, wenn keine Zuschlagstoffe zugesetzt werden. lm Falle der ABA-Triblock-Copolymeren ist im Verhältnis zu den PLGA-Mikropartikeln bei gleichem Beladungsgrad eine verlängerte Freisetzungsdauer festzustellen. Die Freisetzungsdauer verlängert sich bei einem höheren Gehalt von GA im ABA-Triblock-Copolymeren (s.o. längere Freisetzung bei steigendem Gehalt von GA von 11, 16 bzw. 22 Gew.-%).

### Beispiel 5:

In der folgenden Tabelle 5 werden die chemischen und physikalischen Eigenschaften einiger ABA-Blockpolymere in einer Übersicht zusammengestellt.

**Tabelle 5:**

| Übersicht der verwendeten ABA-Triblock-Copolymere | | | | |
|---|---|---|---|---|
| Charge | LA/GA/PEG % | MW [Da] | Tg [°C] | Polydispersität |
| 1 | 64/13/23 | 25.000 | 47.9 | 3.1 |
| 2 | 57/11/32 | 19.000 | 46.3 | 2.5 |
| 3 | 50/12/38 | 20.000 | 46.1 | 2.3 |
| 4 | 45/9/46 | 17.000 | 42.1 | 2.8 |
| 5 | 36/35/29 | 16.400 | 35.3 | 6.8 |
| 6 | 46/22/32 | 16.700 | 45.1 | 5.4 |
| 7 | 42/28/30 | 17.200 | 31.1 | 8.4 |
| 8 | 51/16/33 | 18.500 | 47.9 | 4.6 |
| 9 | 40/20/40 | 13.500 | 23.7 | 5.2 |

Die aus den in Tab. 5 angegebenen ABA-Triblock-Copolymeren hergestellten EPO-haltigen MP wiesen Glasübergangstemperaturen (Tg) im Bereich von 27-45°C auf. Damit ist eine langfristige stabile Lagerung der MP im Kühlschrank (4-8°C) möglich.

### Beispiel 6:

Herstellung von Mikropartikeln unter Kühlung zur Reduktion der Aggregatbildung

Die Herstellung der Mikropartikel erfolgte wie in Beispiel 1 beschrieben mit folgenden Änderungen:
Alle Lösungen (Dichlormethan-Polymer-Lösung, EPO-Lösung in Natrium-Phosphat-Puffer und PVA-Lösung) wurden in einem Eisbad (0 °C) im Kühlraum vorgekühlt. Alle Gefäße und Geräte (z.B. Ultrathurrax) wurden im Kühlraum (4 °C) vorgekühlt. Alle Schritte zur Herstellung der Mikropartikel, inklusive des Rührens der w/o/w-Emulsion zur Härtung der Mikropartikel und zur Evoparation des Dichlormethans wurden im Eisbad im Kühlraum durchgeführt. Die Messung der Temperatur in der 0,1 %igen PVA-Lösung nach Herstellung der w/o/w-Emulsion ergab 1 °C.
Die so hergestellten Mikropartikel hatten folgende vorteilhaften Eigenschaften: Der praktische Beladungsgrad von EPO war größer als beim Standardverfahren (0,54 % statt 0,4 %). Der Aggregatgehalt in Mikropartikeln ohne stabilisierende Zuschlagstoffe war geringer (2 - 5 % statt 10 - 20 %). Der Aggregatgehalt wurde dabei analog dem in Beispiel 2 beschriebenen Verfahren bestimmt.

### Abkürzungsverzeichnis:

- MP:: Mikropartikel
- PLGA:: Copolymer aus Milchsäure und Glykolsäure
- LA:: Milchsäure (lactic acid)
- GA:: Glykolsäure (glycolic acid)
- ABA:: Tripel-Blockcopolymere aus A- und B-Block
- A-Block:: Copolymer aus Milch- und Glykolsäure
- B-Block:: Polyethylenglykol (PEG)

- BSA:: bovines Serumalbumin
- HSA: humanes Serumalbumin
- PVA: Polyvinylalkohol
- D40: Dextran 40.000

## Patentansprüche

1. Pharmazeutische Darreichungsformen in Form von Mikropartikeln bestehend aus einer einen Wirkstoff enthaltenden Polymermatrix, wobei das Polymer ein ABA-Triblock-Copolymer ist, dessen A-Block ein Copolymer aus Milch- und Glykolsäure und dessen B-Block eine Polyethylenglykol-Kette darstellt, dadurch gekennzeichnet, daß der Wirkstoff ein aggregationsempfindliches Polypeptid ist und die Mikropartikel Zuschlagstoffe enthalten, die ausgewählt sind aus der Gruppe Serumproteine, Polyaminosäuren, Cyclodextrine; Cyclodextrinderivate; Saccharide; Aminozucker; Aminosäuren; Detergenzien oder Carbonsäuren sowie Gemische dieser Zuschlagstoffe.

2. Pharmazeutische Darreichungsformen gemäß Anspruch 1, dadurch gekennzeichnet, daß die Zuschlagstoffe ausgewählt sind aus der Gruppe Serumalbumin; Polyaminosäuren; Aminosäuren; Saccharide; Cyclodextrinderivate und Detergenzien.

3. Pharmazeutische Darreichungsformen gemäß Anspruch 1, dadurch gekennzeichnet, daß die Zuschlagstoffe ausgewählt sind aus Gemischen von Dextranen und Polyaminosäuren; Gemischen aus Cyclodextrinen oder Cyclodextrinderivaten mit Aminosäuren; Gemischen von Cyclodextrin oder Cyclodextrinderivaten mit Polyaminosäuren; Gemischen von Cyclodextrinen oder Cyclodextrinderivaten mit Dextranen; oder aus Gemischen von Dextranen mit Aminosäuren.

4. Pharmazeutische Darreichungsformen gemäß einem der Ansprüche 1 - 3, dadurch gekennzeichnet, daß als Zuschlagstoffe in der Polymerphase Carbonsäuren enthalten sind.

5. Darreichungsformen gemäß einem der Ansprüche 1 bis 4, dadurch gekennzeichnet, daß das ABA-Blockpolymer ein Molekulargewicht zwischen 5.000 bis 50.000 Dalton, vorzugsweise zwischen 10.000 bis 30.000, aufweist.

6. Darreichungsformen gemäß einem der Ansprüche 1 bis 5, dadurch gekennzeichnet, daß der Polyethylenglykolanteil im ABA-Copolymer zwischen 20 bis 50 Gew.-%, bezogen auf die gesamte Polymermenge, vorzugsweise zwischen 30 bis 40 Gew.-% liegt.

7. Darreichungsformen gemäß einem der Anprüche 1 bis 6, dadurch gekennzeichnet, daß das Verhältnis von Milchsäure zu Glykolsäure im ABA-Copolymer zwischen 1:1 bis 5:1 liegt, vorzugsweise zwischen 1,5:1 bis 4,5:1.

8. Darreichungsformen gemäß Anspruch 7, dadurch gekennzeichnet, daß das Verhältnis von Milchsäure zu Glykolsäure etwa 4 : 1,vorzugsweise 2 : 1 beträgt.

9. Darreichungsformen gemäß einem der Ansprüche 1 bis 8, dadurch gekennzeichnet, daß die Zuschlagstoffe in einer Menge von 0,5 bis 40 Gew.-%, bezogen auf die Gesamtmikropartikelmenge, vorzugsweise von 1 - 30 Gew.-%, enthalten sind.

10. Darreichungsformen gemäß einem der Ansprüche 1 bis 9, dadurch gekennzeichnet, daß sie als Polyaminosäuren Polyarginin oder Polyhistidin, insbesondere Poly-L-Arginin oder Poly-L-Histidin, enthalten, als Aminosäuren Arginin, Glycin, Phenylalanin, Glutaminsäure oder Lysin enthalten, als Saccharide, Trehalose, Saccharose, Maltose, Stärke, Maltodextrine, Raffinose, Alginat, Dextrane enthalten, als Aminozucker Chitosan oder als Detergenzien oder Triglyceride Tween 20® , Tween 80® , Pluronic® oder Miglyol® enthalten.

11. Darreichungsformen gemäß Anspruch 10, dadurch gekennzeichnet, daß sie als Dextrane solche mit einem Molekulargewicht zwischen 20.000 und 60.000, vorzugsweise 40.000, enthalten.

12. Darreichungsformen gemäß einem der Ansprüche 1 bis 11, dadurch gekennzeichnet, daß sie als Zuschlagstoffe Gemische aus Dextranen mit Polyarginin oder Polyhistidin enthalten, vorzugsweise Gemische aus Dextran 40.000 mit Poly-L-Arginin oder Gemische aus Dextran 40.000 mit Poly-L-Histidin.

13. Darreichungsformen gemäß einem der Ansprüche 1-9, dadurch gekennzeichnet, daß sie als Zuschlagstoffe Serumproteine enthalten, vorzugsweise bovines oder humanes Serumalbumin.

14. Darreichungsformen gemäß einem der Ansprüche 1 bis 13, dadurch gekennzeichnet, daß der Polypeptidgehalt in den Mikropartikeln zwischen 0,01% bis zu 5 Gew.-%, bezogen auf die Gesamtmikropartikelmenge, vorzugsweise zwischen 0,01% bis zu 3 Gew.-% beträgt.

15. Darreichungsformen gemäß einem der Ansprüche 1 bis 14, dadurch gekennzeichnet, daß sie Erythropoietin als Polypeptid enthalten.

16. Verfahren zur Herstellung von Polypeptid-enthaltenden Mikropartikeln mit Hilfe des Tripelemulsionsverfahrens, umfassend die Schritte:
a) Lösen eines ABA-Triblock-Copolymeren, dessen A-Block ein Copolymer aus Milch- und Glykolsäure ist und dessen B-Block ein Polyethylenglykol-Kette darstellt, in einem organischen, nicht mit Wasser mischbaren Lösungsmittel, gegebenenfalls unter Zusatz einer Carbonsäure;
b) Zugabe einer Lösung oder einer Suspension eines Polypeptides, wobei die Lösung oder Suspension weitere Zuschlagstoffe enthält, die ausgewählt sind aus der Gruppe Serumproteine, Polyaminosäuren; Cyclodextrine; Cyclodextrinderivate; Saccharide; Aminozucker; Aminosäuren, Detergenzien sowie Gemische dieser Zuschlagstoffe, und Herstellung einer W/O-Emulsion in einem ersten Homogenisierungsschritt;
c) Herstellen einer W/O/W-Emulsion in einem zweiten Homogenisierungsschritt durch Dispersion der erhaltenen W/O-Emulsion in einer Stabilisator-haltigen wässrigen Lösung; und
d) Verdunsten des Lösungsmittels und anschließender Isolierung der Mikropartikel.

17. Verfahren nach Anspruch 16, dadurch gekennzeichnet, daß im ersten Homogenisierungsschritt als Homogenisator ein Ultra-Turrax eingesetzt wird und einmal 30 Sekunden oder zweimal 30 Sekunden, mit einer Pause von 30 Sekunden dazwischen, dispergiert wird.

18. Verfahren nach Anspruch 16 oder 17, dadurch gekennzeichnet, daß ein Gewichtsverhältnis Wasser/organische Phase von bis zu 20 - 25% gewählt wird.

19. Verfahren nach einem der Ansprüche 16 - 18, dadurch gekennzeichnet, daß der gesamte Herstellungsprozeß bei Temperaturen zwischen 0 und 6 °C durchgeführt wird.

20. Verwendung von pharmazeutischen Zuschlagstoffen ausgewählt aus der Gruppe Serumproteine, Polyaminosäuren; Cyclodextrine; Cyclodextrinderivate; Saccharide; Aminozucker; Aminosäuren; Detergenzien oder Carbonsäuren sowie Gemische dieser Zuschlagstoffe zur Vermeidung der Aggregatbildung von aggregationsempfindlichen Polypeptiden bei der Herstellung von Polypeptid-enthaltenden Mikropartikeln.

## Claims

1. Pharmaceutical forms of administration in the form of microparticles composed of a polymer matrix containing an active substance, the polymer being an ABA triblock copolymer, the A block of which is a copolymer composed of lactic and glycolic acid, and the B block of which represents a polyethylene glycol chain, wherein the active substance is an aggregation-sensitive polypeptide, and the microparticles contain additives which are selected from the group comprising serum proteins, polyamino acids, cyclodextrins, cyclodextrin derivatives, saccharides, amino sugars, amino acids, detergents or carboxylic acids, as well as mixtures of these additives.

2. Pharmaceutical forms of administration as claimed in claim 1, wherein the additives are selected from the group comprising serum albumin, polyamino acids, amino acids, saccharides, cyclodextrin derivatives, and detergents.

3. Pharmaceutical forms of administration as claimed in claim 1, wherein the additives are selected from mixtures of dextrans and polyamino acids, mixtures of cyclodextrins or cyclodextrin derivatives with amino acids, mixtures of cyclodextrin or cyclodextrin derivatives with polyamino acids, mixtures of cyclodextrins or cyclodextrin derivatives with dextrans or of mixtures of dextrans with amino acids.

4. Pharmaceutical forms of administration as claimed in one of the claims 1 - 3, wherein carboxylic acids are contained in the polymer phase as additives.

5. Forms of administration as claimed in one of the claims 1 to 4, wherein the ABA block polymer has a molecular weight of between 5,000 and 50,000 Daltons, preferably between 10,000 and 30,000.

6. Forms of administration as claimed in one of the claims 1 to 5, wherein the proportion of polyethylene glycol in the ABA copolymer is between 20 and 50% by weight relative to the total amount of polymer, preferably between 30 and 40% by weight.

7. Forms of administration as claimed in one of the claims 1 to 6, wherein the ratio of lactic acid to glycolic acid in the ABA copolymer is between 1:1 and 5:1, preferably between 1.5:1 and 4.5:1.

8. Forms of administration as claimed in claim 7, wherein the ratio of lactic acid to glycolic acid is about 4:1, preferably 2:1.

9. Forms of administration as claimed in one of the claims 1 to 8, wherein the additives are present in an amount of 0.5 to 40% by weight relative to the total amount of microparticles, preferably of 1-30% by weight.

10. Forms of administration as claimed in one of the claims 1 to 9, wherein they contain polyarginine or polyhistidine, in particular poly-L-arginine or poly-L-histidine as polyamino acids, they contain arginine, glycine, phenylalanine, glutamic acid, or lysine as amino acids, they contain trehalose, sucrose, maltose, starch, maltodextrin, raffinose, alginate, dextrans as saccharides, chitosan as an amino sugar, Tween 20® , Tween 80® , Pluronic® , or Miglyol® as detergents or triglycerides.

11. Forms of administration as claimed in claim 10, wherein they contain dextrans with a molecular weight between 20,000 and 60,000, preferably 40,000.

12. Forms of administration as claimed in one of the claims 1 to 11, wherein they contain mixtures of dextrans with polyarginine or polyhistidine as additives, preferably mixtures of dextran 40,000 with poly-L-arginine or mixtures of dextran 40,000 with poly-L-histidine.

13. Forms of administration as claimed in one of the claims 1-9, wherein they contain serum proteins as the additives, preferably bovine serum albumin or human serum albumin.

14. Forms of administration as claimed in one of the claims 1 to 13, wherein the content of polypeptide in the microparticles is between 0.01% and up to 5% by weight relative to the total amount of microparticles, preferably between 0.01% and up to 3% by weight.

15. Forms of administration as claimed in one of the claims 1 to 14, wherein they contain erythropoietin as the polypeptide.

16. Process for the production of microparticles containing polypeptide with the aid of the triple emulsion process comprising the steps:
a) dissolving an ABA triblock copolymer, the A block of which is a copolymer composed of lactic and glycolic acid, and the B block of which represents a polyethylene glycol chain in an organic, water-immiscible solvent optionally with addition of a carboxylic acid;
b) adding a solution or a suspension of a polypeptide, wherein the solution or suspension contains further additives which are selected from the group comprising serum proteins, polyamino acids, cyclodextrins, cyclodextrin derivatives, saccharides, amino sugars, amino acids, detergents, as well as mixtures of these additives and producing a W/O emulsion in a first homogenization step;
c) producing a W/O/W emulsion in a second homogenization step by dispersion of the W/O emulsion obtained in an aqueous solution containing stabilizer; and
d) evaporating the solvent and subsequently isolating the microparticles.

17. Process as claimed in claim 16, wherein in a first homogenization step an Ultra Turrax is used as the homogenizer, and it is dispersed once for 30 seconds or twice for 30 seconds with an interval of 30 seconds in between.

18. Process as claimed in claim 16 or 17, wherein a weight ratio of water/organic phase of up to 20-25% is selected.

19. Process as claimed in one of the claims 16-18, wherein the entire production process is carried out at temperatures between 0 and 6°C.

20. Use of pharmaceutical additives selected from the group comprising serum proteins, polyamino acids, cyclodextrins, cyclodextrin derivatives, saccharides, amino sugars, amino acids, detergents, or carboxylic acids, as well as mixtures of these additives to avoid aggregate formation of aggregation-sensitive polypeptides during the production of microparticles containing polypeptides.

## Revendications

1. Formes pharmaceutiques d'administration sous forme de microparticules constituées d'une matrice polymère contenant une substance active, le polymère étant un copolymère triséquencé ABA, dont la séquence A représente un copolymère d'acide lactique et d'acide glycolique et dont la séquence B représente une chaine de polyéthylèneglycol, caractérisées en ce que la substance active est un polypeptide sensible à l'agrégation et les microparticules contiennent des additifs qui sont choisis dans le groupe des protéines sériques, polyaminoacides, cyclodextrines, dérivés de cyclodextrines, saccharides, aminosaccharides, aminoacides, détergents ou acides carboxyliques, ainsi que des mélanges de ces additifs.

2. Formes pharmaceutiques d'administration selon la revendication 1, caractérisées en ce que les additifs sont choisis dans l'ensemble constitué par l'albumine sérique, des polyaminoacides, aminoacides, saccharides, dérivés de cyclodextrine et détergents.

3. Formes pharmaceutiques d'administration selon la revendication 1, caractérisées en ce que les additifs sont choisis parmi des mélanges de dextranes et de polyaminoacides, des mélanges de cyclodextrines ou de dérivés de cyclodextrines avec des aminoacides, des mélanges de cyclodextrine ou de dérivés de cyclodextrines avec des polyaminoacides, des mélanges de cyclodextrines ou de dérivés de cyclodextrines avec des dextranes, ou des mélanges de dextranes avec des aminoacides.

4. Formes pharmaceutiques d'administration selon l'une des revendications 1 à 3, caractérisées en ce que des acides carboxyliques sont contenus en tant qu'additifs dans la phase de polymère.

5. Formes pharmaceutiques d'administration selon l'une des revendications 1 à 4, caractérisées en ce que le copolymère séquencé ABA présente une masse moléculaire comprise entre 5 000 et 50 000 daltons, de préférence, entre 10 000 et 30 000 Da.

6. Formes pharmaceutiques d'administration selon l'une des revendications 1 à 5, caractérisées en ce que la proportion du polyéthylèneglycol dans le copolymère ABA est comprise entre 20 et 50 % en poids, de préférence, entre 30 et 40 % en poids, par rapport à la quantité totale de polymère.

7. Formes pharmaceutiques d'administration selon l'une des revendications 1 à 6, caractérisées en ce que le rapport de l'acide lactique à l'acide glycolique dans le copolymère ABA est compris entre 1:1 et 5:1, de préférence, entre 1,5:1 et 4,5:1.

8. Formes pharmaceutiques d'administration selon la revendication 7, caractérisées en ce que le rapport de l'acide lactique à l'acide glycolique est d'environ 4:1, de préférence 2 :1.

9. Formes pharmaceutiques d'administration selon l'une des revendications 1 à 8, caractérisées en ce que les additifs sont contenus en une proportion de 0,5 à 40 % en poids, de préférence, de 1 à 30% en poids, par rapport à la quantité totale de microparticules.

10. Formes pharmaceutiques d'administration selon l'une des revendications 1 à 9, caractérisées en ce qu'elles contiennent en tant que polyaminoacides de la polyarginine ou de la polyhistidine, en particulier de la poly-L-arginine ou de la poly-L-histidine, en tant qu'aminoacides de l'arginine, de la glycine, de la phénylalanine, de l'acide glutamique ou de la lysine, en tant que saccharides, du tréhalose, du saccharose, du maltose, de l'amidon, de la maltodextrine, du raffinose, un alginate, des dextranes, en tant qu'aminosaccharides, du chitosane, ou, en tant que détergents ou triglycérides, du Tween 20® , du Tween 80® , du Pluronic® ou du Miglyol® .

11. Formes pharmaceutiques d'administration selon la revendication 10, caractérisées en ce qu'elles contiennent en tant que dextranes de tels dextranes ayant une masse moléculaire comprise entre 20 000 et 60 000, de préférence, de 40 000.

12. Formes pharmaceutiques d'administration selon l'une des revendications 1 à 11, caractérisées en ce qu'elles contiennent en tant qu'additifs des mélanges de dextranes avec de la polyarginine ou de la polyhistidine, de préférence, des mélanges de dextrane 40 000 avec de la poly-L-arginine ou des mélanges de dextrane 40 000 avec de la poly-L-histidine.

13. Formes pharmaceutiques d'administration selon l'une des revendications 1 à 9, caractérisées en ce qu'elles contiennent en tant qu'additifs des protéines sériques, de préférence, de l'albumine sérique bovine ou humaine.

14. Formes pharmaceutiques d'administration selon l'une des revendications 1 à 13, caractérisées en ce que la proportion des polypeptides dans les microparticules est comprise entre 0,01 % et 5 % en poids, de préférence, entre 0,01 % et 3 % en poids, par rapport à la quantité totale de microparticules.

15. Formes pharmaceutiques d'administration selon l'une des revendications 1 à 14, caractérisées en ce qu'elles contiennent en tant que polypeptide de l'érythropoïétine.

16. Procédé pour la préparation de microparticules contenant des polypeptides, à l'aide du procédé d'émulsion triple, comprenant les étapes suivantes:
a) dissolution d'un copolymère triséquencé ABA, dont la séquence A représente un copolymère d'acide lactique et d'acide glycolique et dont la séquence B représente une chaîne de polyéthylèneglycol, dans un solvant organique non miscible à l'eau, éventuellement avec addition d'un acide carboxylique ;
b) addition d'une solution ou d'une suspension d'un polypeptide, la solution ou la suspension contenant d'autres additifs qui sont choisis dans le groupe des protéines sériques, polyaminoacides, cyclodextrines, dérivés de cyclodextrines, saccharides, aminosaccharides, aminoacides, détergents ainsi que des mélanges de ces additifs, et préparation d'une émulsion E/H dans une première étape d'homogénéisation ;
c) préparation d'une émulsion E/H/E dans une seconde étape d'homogénéisation par dispersion de l'émulsion E/H obtenue, dans une solution aqueuse contenant un stabilisant ; et
d) évaporation du solvant et isolement subséquent des microparticules.

17. Procédé selon la revendication 16, caractérisé en ce que, dans une première étape d'homogénéisation, on utilise comme homogénéisateur un homogénéisateur Ultra-Turrax et on effectue la dispersion une fois pendant 30 secondes ou deux fois pendant 30 secondes avec une pause de 30 secondes entre les deux.

18. Procédé selon la revendication 16 ou 17, caractérisé en ce que l'on choisit un rapport pondéral eau/phase organique allant jusqu'à 20-25 %.

19. Procédé selon l'une des revendications 16 à 18, caractérisé en ce que le processus de préparation total est effectué à des températures comprises entre 0 et 6°C.

20. Utilisation d'additifs pharmaceutiques choisis dans le groupe des protéines sériques, polyaminoacides, cyclodextrines, dérivés de cyclodextrines, saccharides, aminosaccharides, aminoacides, détergents ou acides carboxyliques ainsi que des mélanges de ces additifs, pour éviter la formation d'agrégats de polypeptides sensibles à l'agrégation, dans la préparation de microparticules contenant des polypeptides.
